# EUROPEAN PATENT APPLICATION

(11) **EP 3 715 453 A1**
(43) Date of publication of application: **30.09.2020**
(21) Application number: 19165086.0
(22) Date of filing: 26.03.2019
(51) Int. Cl.: C12N 7/00, C12N 9/22, C12N 15/10

(54) **METHODS AND PRODUCTS FOR A SAFE AND EFFICIENT TRANSDUCTION OF GENE EDITING COMPONENTS**

(71) Applicant: Johann Wolfgang Goethe-Universität Frankfurt, 60323 Frankfurt am Main (DE); Deutsches Krebsforschungszentrum, Stiftung des öffentlichen Rechts, 69120 Heidelberg (DE)
(72) Inventor: Serve, Hubert, 60431 Frankfurt am Main (DE); Schnütgen, Frank, 65719 Hofheim am Taunus (DE); Gatzke, Florian, 61137 Schöneck (DE)
(74) Representative: Kuttenkeuler, David

(57) **Abstract**

The invention is based on the surprising finding of the ability of gene editing nucleases to be automatically packaged into viral particles. The invention provides viral particles, such as viral capsids, containing a high amount of the gene editing nuclease that efficiently transduce target cells with either components for gene editing or alternatively any other cargo attached to the gene editing nuclease. Since the viral particles of the invention do not need to contain any components of the viral genome, they can both efficiently and safely transduce mammalian cells for editing genome sequences or other purposes. The invention provides the viral particles, compositions comprising them, methods for their generation, and medical and laboratory applications of the new transduction methods and tools.

## Description

### FIELD OF THE INVENTION

The invention is based on the surprising finding of the ability of gene editing nucleases to be automatically packaged into viral particles. The invention provides viral particles, such as viral capsids, containing a high amount of the gene editing nuclease that efficiently transduce target cells with either components for gene editing or alternatively any other cargo attached to the gene editing nuclease. Since the viral particles of the invention do not need to contain any components of the viral genome, they can both efficiently and safely transduce mammalian cells for editing genome sequences or other purposes. The invention provides the viral particles, compositions comprising them, methods for their generation, and medical and laboratory applications of the new transduction methods and tools.

### DESCRIPTION

CRISPR / Cas9 gene editing has revolutionized the modification of genomic DNA through its unsurpassed ease of use ^{[1]}. Despite its success in preclinical research, the system to date has not been successfully applied in the clinic therapeutically. Currently, there are only 17 clinical trials worldwide that investigate the therapeutic use and potential of a lentiviral inserted CRISPR / Cas9 system. One problem with the route of delivery via integrating lentiviruses is the low efficiency, which is a consequence of inefficient transduction and expression of CRISPR / Cas9 protein due to the size of the vector construct. Accordingly, the amount of transducing units (TU) must be very high in order to ensure efficiency. This circumstance increases the risk of insertional mutagenesis in the host genome, with chances of carcinogenesis. Prolonged expression of CRISPR / Cas9 as a result of stable integration into the host cell genome further increases the frequency of unintended and uncontrolled gene modifications distant to the targeted genetic locus ^{[2]}.

Integrase-deficient lentiviruses have been developed as an alternative route of administration for clinical gene editing. In this case, a mutation of the viral integrase molecule prevents incorporation into the host genome. However, studies have demonstrated residual integration events with a probability of approximately 5% of all transduced cells ^{[3]}. As a non-integrative viral alternative to the transduction of target cells, adeno-associated viruses were used. However, the systems reported so far do not achieve sufficient therapeutic efficacy: the reason is the long coding sequence of the CRISPR / Cas9 molecule, which exceeds the limit of codable genetic information in adeno-associated viruses. As a solution to this problem, the so-called split / Cas9 derivative was developed ^{[4]}: Here, the coding sequence of CRISPR / Cas9 was encoded on two separate adenoviruses. The resulting protein fragments combine in the target cell to form a functional CRISPR/Cas9 molecule. The system therefore requires a double transduction to be functional, which in reality limits gene editing efficiency. Another alternative is the use of a CRISPR / Cas9 *Staphylococcus aureus* strain in order to identify an orthologous but smaller CRISPR / Cas9 species whose coding sequence would fit into the coding limit of adenoviruses ^{[5]}. The time-constricted introduction of CRISPR / Cas9 ribonucleoproteins by electroporation as a further option allows efficient gene modification with a reduction in off-target effects ^{[6]}. Disadvantages are the limitation of the clinical applicability to the field of hematology as well as the method-associated risk of causing a plethora of damages within the target cell as a consequence of the high electrical currents and associated magnetic fields. Long-term results on therapeutic applications of electroporated cells are still completely lacking, so that the system cannot be sufficiently evaluated with regard to its therapeutic safety profile.

In order to combine the advantages of the viral biological, non-toxic route of administration with the limited temporal presence of CRISPR / Cas9 ribonucleoproteins, it has been shown that it is technically possible to package CRISPR / Cas9 ribonucleoproteins into retroviral virions by fusing CRISPR / Cas9 proteins via an HIV protease cleavage site to viral capsid proteins. However, HIV proteases mediate a functional but inefficient proteolysis-based release of CRISPR / Cas9 ribonucleoproteins from the virions and thus the system still remains without appreciable therapeutic efficacy ^{[7]}. Montagna et al. recently reported that co-transfection of the viral envelope protein VGV-G and an expression plasmid for CRISPR / Cas9 and and sgRNA led to the secretion of CRISPR / Cas9 ribonucleorotein into vesicles, which can be used to edit genetic information, but have limited effectiveness in comparison to our invention^{[8]}. Recently developed non-viral transduction systems for Cas9 Ribonucleoproteins do not allow for targeting a specific cell type.

### BRIEF DESCRIPTION OF THE INVENTION

Generally, and by way of brief description, the main aspects of the present invention can be described as follows:
In **a first aspect,** the invention pertains to a viral particle for delivering cargo into a cell and/or into a cell nucleus, the viral particle comprising a first particle coat encasing a first particle volume, wherein the first particle volume comprises the cargo compound, and a shuttle compound, the shuttle compound comprising a gene-editing nuclease protein, or a fragment or derivative thereof.
In **a second aspect,** the invention pertains to a method for producing a viral particle for use in the delivery of a cargo compound into a cell and/or into a cell nucleus, the method comprising the step of
   (a) Introducing into a viral packaging cell one or more genetic constructs for the expression of a shuttle compound and a cargo compound, wherein the one or more genetic constructs comprise (i) a first expressible sequence encoding a gene-editing nuclease protein, or a derivative or fragment thereof, and (ii) a second expressible sequence encoding a cargo compound; alternatively, wherein (i) is not introduced into the viral packaging cell but provided as a stable, preferably genome integrated, expression construct in said viral packaging cell;
   (b) Expressing the first and the second expressible sequences in the viral packaging cell,
   (c) Expressing viral particle components for viral packaging in the viral packaging cell and thereby packaging the shuttle compound and the cargo compound into the viral particle, and
   (d) Harvesting the so produced viral particle.
In **a third aspect,** the invention pertains to a method for producing a viral particle for use in the delivery of gene-editing components into a target cell and/or into a target cell nucleus, the method comprising the step of
   (a) Introducing into a viral packaging cell one or more genetic constructs for the expression of gene-editing components, wherein the one or more genetic constructs comprise (i) a first expressible sequence encoding a gene-editing nuclease, or a derivative or fragment thereof and (ii) a second expressible sequence encoding at least for one gene-editing guide nucleic acid; alternatively, wherein (i) is not introduced into the viral packaging cell but provided as a stable, preferably genome integrated, expression construct in said viral packaging cell;
   (b) Expressing the first and the second expressible sequences in the viral packaging cell,
   (c) Expressing viral particle components for viral packaging in the viral packaging cell, and
   (d) Harvesting the so produced viral particle.
In **a fourth aspect,** the invention pertains to a viral particle produced with a method according to the second or third aspect of the invention.
In **a fifth aspect,** the invention pertains to a composition comprising a viral particle according to the first or fourth aspect of the invention, together with a carrier and/or excipient.
In **a sixth aspect,** the invention pertains a product for use in the treatment of a disease, wherein the product is a viral particle according to the first or fourth aspect of the invention, or is a composition according to the fifth aspect of the invention.
In **a seventh aspect,** the invention pertains an *in vitro* use of (i) a viral particle according to the first or fourth aspect of the invention, or (ii) of a composition according to the fifth aspect of the invention, in the targeted delivery of one or more cargo compounds into a target cell and/or into a target cell nucleus.
In **an eighth aspect,** the invention pertains to a method of editing a genomic sequence in a target cell, comprising a step of contacting (or transducing) the target cell and/or a target cell nucleus, with a viral particle according to the first or fourth aspect of the invention, or with a composition according to the fifth aspect of the invention.
In **a ninth aspect,** the invention pertains to A system for editing a target genome, the system comprising
   (i) a genetic expression construct comprising an expressible sequence of a gene-editing nuclease, or a fragment or derivative thereof,
   (ii) genetic expression construct comprising an expressible expression cassette for introducing a sequence of a gene-editing guide nucleic acid sequence,
   wherein the genetic construct of (i) an (ii) can be provided on one nucleic acid molecule or separate nucleic acid molecules, and wherein the genetic construct(s) is(are) not able to integrate into a target genome.
In **a tenth aspect,** the invention pertains to a kit of parts comprising one or more, or all of, the components of the system according to the ninth aspect of the invention.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect,** the invention pertains to a viral particle for delivering cargo into a cell and/or into a cell nucleus, the viral particle comprising a first particle coat encasing a first particle volume, wherein the first particle volume comprises the cargo compound, and a shuttle compound, the shuttle compound comprising a gene-editing nuclease protein, or a fragment or derivative thereof. In accordance with the herein disclosed invention it was surprisingly discovered that gene editing nuclease proteins, in particular CRISPR / Cas9 proteins, or fragments or derivatives thereof, have the ability to be packaged into viral particles without the need of a viral genome to be present in the particle. Hence, the present invention provides the application of the packaging of such proteins into viral particles without the need of any attachment or coupling to viral components. The packaging therefore, in some embodiments, proceeds by simple expression of the gene editing nuclease protein, or fragment or derivatives thereof, in a cell that also expresses all necessary proteins to form a viral particle.

The term "viral particle" shall denote any virus derived body composed of a proteinaceous coat (first particle coat) which encases a volume (first particle volume). Such particles may further comprise additional coat layers encasing a core viral particle. Between the first and the second layer therefore an additional particle volume (second particle volume) exists. Complexes viruses for example are composed of a proteinaceous capsid particle that is encased by a second coating lipid bilayer. Sizes and composition of the viral particle may vary significantly depending on from which virus they are derived. However, preferred are viral particles derived from lentivirus (or also other retroviruses). In context of the present invention each particle surface layer is referred to as a "coat". Hence, in some embodiments of the invention the first particle coat is a viral capsid, such as a surface composed of viral capsid proteins. The term "viral capsid protein" refers to a protein of any virus that comprises a shell around its genome, and therefore is different from a "viral envelope protein" which refers to any viral protein that covers a capsid protein shell and becomes part of the outer layer of a virus.

In preferred embodiments of the invention the first particle coat does not comprise a lipid mono- or bilayer, preferably wherein the viral particle is not an exosome, liposome, micelle or other particle essentially composed of amphiphilic lipids. Hence, as mentioned above, in such preferred embodiments the first viral particle coat of the invention is further preferably a viral capsid. In some preferred embodiments the gene-editing nuclease protein is essentially absent from the first particle coat. "Essentially absent" in this context shall denote embodiments where the bulk amount of gene editing nuclease protein is not located within the coat layer but within the volume it encases. In this aspect more than 50% of total gene editing nuclease is located within the first particle volume and not the first particle coat layer, more preferably 90%, or 95%. Furthermore, in additional or alternative embodiments, the gene editing nuclease protein is not fused to a structural component of the first particle coat, preferably wherein such structural component is a protein, such as a viral coat protein. Preferably, "not fused" shall mean that the gene editing nuclease protein is not covalently attached to the first particle coat. However, in the process of packaging the gene editing nuclease into the first particle volume in a packaging cell, it might occur that the gene editing nuclease protein gets associated non-covalently, and not via any specifically introduced linker proteins, with the first particle coat or coat proteins. The bulk amount of gene editing nuclease protein however in the viral particle of the invention is packaged within the first particle volume in preferred embodiments. The viral particle according to the invention, thus, in some embodiments further comprises a second particle coat encasing the first particle coat within an intermediate (second) particle volume. The second particle coat is herein also referred to as envelope and in preferred embodiments is composed of a lipid bilayer, and wherein the first particle coat forms a viral capsid. Hence, such a viral particle is preferably a retrovirus derived particle, preferably a lentivirus derived particle.

The viral particle of the invention in preferred embodiments essentially does not comprise a functional integrase, preferably essentially not comprising a functional integrase protein and essentially not comprising an expressible and/or translatable nucleic acid sequence encoding for a functional integrase protein. An integrase (retroviral integrase) is an enzyme produced by a retrovirus (such as a lentivirus) that enables its genetic material to be integrated into the DNA of the infected cell. A non-functional integrase in accordance with the present invention is therefore an integrase protein that is mutated such that it cannot perform its original function in the viral life cycle, and in particular cannot mediate or catalyse the introduction of genetic material into a target genome. In preferred embodiments the viral particle of the invention does not comprise an integrase protein or any fragment or derivative of such a molecule.

In further preferred embodiments, the viral particle of the invention does essentially not comprise a full or partial viral genome. In this embodiment the viral particle does not comprise any genetic information of a virus.

The gist of the present invention can be seen in the fact that a gene editing nuclease as described herein is packaged virtually automatically into a viral capsid by a viral packaging cell line, or a cell line expressing the necessary structural proteins for producing viral particles. Therefore, other than prior art approaches, in the viral particle of the invention the first particle coat, or a structural component of the first particle coat, essentially does not comprise, or essentially does not contain, or is not covalently fused to, a gene-editing nuclease protein. Hence, in accordance with the invention no modifications of a gene editing nuclease protein is necessary nor intended that facilitates the packaging of the gene editing nuclease protein into a viral particle.

In some embodiments, which are preferred, the viral particle of the invention may comprise a particle surface-presented targeting molecule suitable for targeting the delivery of the particle to a target cell type. Depending on the viral particle, the surface-presented targeting molecule is located on the first, second or further particle coat, but preferably is always on the most outside surface of the particle, or at least on such a surface which will be recognized by a target cell. Such particle surface-presented targeting molecule is preferably a protein, such as an antibody-like protein or DARPin or targeting ligand peptide identified from phage- or AAV display libraries. Examples include CD8 or CD4 antibodies targeting viral infections specifically to T-cells. Other targets for directing viral delivery are selected from CD8, Cd4, CD19, Cd20, CD33, Cd133, NKp46, endoglin (CD105), GluA4, cd30, Cd46, SLAM, CD340/Her2/neu, Mucin-4,P-Glycoprotein, Prostate stem cell antigen, cd34, CD146, CD326, αvβ8 integrin, PD-1, PD-L1, CTLA-4, CD47, and Siglec15. In context of the herein disclosed invention using a phage or AAV-display technology can identify ligands fur such purposes.

In principle the technology of the herein described invention can be used to target any cell or tissue. However, as examples, the target cell type is preferably a fetal cord blood cell, a fibroblast cell, a brain cell, a lung cell, a liver cell, a muscle cell, a pancreatic cell, an endothelial cell, a cardiac cell, a skin cell, a bone marrow stromal cell, and an eye cells, a pancreatic ductal cell, a neural precursor, or a mesodermal stem cell. Further preferred are bone marrow cells, hematopoietic progenitor cells, or any immunological cell, such as antigen presenting cells (dendritic cells), natural killer cells, lymphocytes, T cells, B cells, macrophages and medullary thymic epithelial cells (mTEC), myeloid derived suppressor cells, T-regulatory cells, as well as stem cells or progenitor cells, hepatic sinusoidal endothelial cells

As an alternative to a full length gene editing nuclease protein of the invention, also fragments or derivatives thereof can be used. Such fragment or derivative of the gene-editing nuclease protein preferably retains the ability of the gene-editing nuclease protein to be packaged into a viral capsid. If the purpose of the use of the technology is to edit a target genome, then such fragment or derivative of the gene editing nuclease protein furthermore retains the any enzymatic activity facilitating the genome editing reation. If the gene editing nuclease protein is merely used as a shuttle to deliver another cargo, for example by covalently attaching the cargo to the automatically packaged gene editing nuclease protein fragment or derivative, then such fragment or derivative merely retains the packaging function, however, not necessarily other enzymatic activities. In such embodiments it might be preferably to reduce the size of the shuttle component by removing certain parts of the gene editing nuclease protein which are not necessary for mediating an automatically packaging of the protein into a viral particle (such as a capsid).

The viral particle according to the invention in some preferred embodiments comprises within the first particle volume at least one species of a gene-editing guide nucleic acid, such as a guide RNA (gRNA), or single guide RNA (sgRNA), or a guide DNA (gDNA) or single guide DNA (sgDNA) or combinations or variants thereof. A "gene editing nucleic acid" in context of the invention shall be any nucleic acid molecule that binds to the gene editing nuclease protein of the invention, and in particular to a Cas9 polypeptide. Such gene editing nucleic acid targets the gene editing nuclease protein to a specific location within the target nucleic acid (target genome) ad is referred to herein as a "guide nucleic acid". When the guide nucleic acid is an RNA molecule, it can be referred to as a "guide RNA" or a "gRNA". A guide nucleic acid comprises two segments, a first segment (referred to herein as a "targeting segment"); and a second segment (referred to herein as a "protein-binding segment"). By "segment" it is meant a segment/section/region of a molecule, e.g., a contiguous stretch of nucleotides in a nucleic acid molecule. A segment can also mean a region/section of a complex such that a segment may comprise regions of more than one molecule. For example, in some cases the protein-binding segment (described below) of a guide nucleic acid is one nucleic acid molecule (e.g., one RNA molecule) and the protein-binding segment therefore comprises a region of that one molecule. In other cases, the protein-binding segment (described below) of a guide nucleic acid comprises two separate molecules that are hybridized along a region of complementarity. As an illustrative, non-limiting example, a protein-binding segment of a guide nucleic acid that comprises two separate molecules can comprise (i) base pairs 40-75 of a first molecule (e.g., RNA molecule, DNA/RNA hybrid molecule) that is 100 base pairs in length; and (ii) base pairs 10-25 of a second molecule (e.g., RNA molecule) that is 50 base pairs in length. The definition of "segment," unless otherwise specifically defined in a particular context, is not limited to a specific number of total base pairs, is not limited to any particular number of base pairs from a given nucleic acid molecule, is not limited to a particular number of separate molecules within a complex, and may include regions of nucleic acid molecules that are of any total length and may or may not include regions with complementarity to other molecules.

The first segment (targeting segment) of a guide nucleic acid (e.g., guide RNA) comprises a nucleotide sequence that is complementary to a specific sequence (a target site) within a target nucleic acid (e.g., a target ssRNA, a target ssDNA, the complementary strand of a double stranded target DNA, etc.). The protein-binding segment (or "protein-binding sequence") interacts with a Cas9 polypeptide. Site-specific binding and/or cleavage of the target nucleic acid can occur at locations determined by base-pairing complementarity between the guide nucleic acid (e.g., guide RNA) and the target nucleic acid. The protein-binding segment of a subject guide nucleic acid comprises two complementary stretches of nucleotides that hybridize to one another to form a double stranded RNA duplex (dsRNA duplex).

A subject guide nucleic acid (e.g., guide RNA) and a subject Cas9 polypeptide form a complex (i.e., bind via non-covalent interactions). The guide nucleic acid (e.g., guide RNA) provides target specificity to the complex by comprising a nucleotide sequence that is complementary to a sequence of a target nucleic acid. The nuclease polypeptide of the complex provides the site-specific activity. In other words, the nuclease polypeptide is guided to a target nucleic acid sequence (e.g. a target sequence in a chromosomal nucleic acid; a target sequence in an extrachromosomal nucleic acid, e.g. an episomal nucleic acid, a minicircle, an RNA, a DNA, etc.; a target sequence in a mitochondrial nucleic acid; a target sequence in a chloroplast nucleic acid; a target sequence in a plasmid; etc.) by virtue of its association with the protein-binding segment of the guide nucleic acid. The associated complex is referred to herein as "ribonucleoprotein".

In some embodiments, a subject guide nucleic acid (e.g., guide RNA) comprises two separate nucleic acid molecules: an "activator" and a "targeter" (see below) and is referred to herein as a "dual guide nucleic acid", a "double-molecule guide nucleic acid", or a "two-molecule guide nucleic acid." If both molecules of a dual guide nucleic acid are RNA molecules, the dual guide nucleic acid can be referred to as a "dual guide RNA" or a "dgRNA." In some embodiments, the subject guide nucleic acid is a single nucleic acid molecule (single polynucleotide) and is referred to herein as a "single guide nucleic acid", a "single-molecule guide nucleic acid," or a "one-molecule guide nucleic acid." If a single guide nucleic acid is an RNA molecule, it can be referred to as a "single guide RNA" or an "sgRNA." The terms "gene editing nucleic acid" or "guide nucleic acid" are inclusive, referring to both dual guide nucleic acids and to single guide nucleic acids (e.g., dgRNAs, sgRNAs, etc.) while the term "guide RNA" is also inclusive, referring to both dual guide RNA (dgRNA) and single guide RNA (sgRNA).

In some cases, a guide nucleic acid is a DNA/RNA hybrid molecule. In such cases, the protein-binding segment of the guide nucleic acid is RNA and forms an RNA duplex. However, the targeting segment of a guide nucleic acid can be DNA. Thus, if a DNA/RNA hybrid guide nucleic acid is a dual guide nucleic acid, the "targeter" molecule and be a hybrid molecule (e.g., the targeting segment can be DNA and the duplex-forming segment can be RNA). In such cases, the duplex-forming segment of the "activator" molecule can be RNA (e.g., in order to form an RNA-duplex with the duplex-forming segment of the targeter molecule), while nucleotides of the "activator" molecule that are outside of the duplex-forming segment can be DNA (in which case the activator molecule is a hybrid DNA/RNA molecule) or can be RNA (in which case the activator molecule is RNA). If a DNA/RNA hybrid guide nucleic acid is a single guide nucleic acid, then the targeting segment can be DNA, the duplex-forming segments (which make up the protein-binding segment) can be RNA, and nucleotides outside of the targeting and duplex-forming segments can be RNA or DNA.

An exemplary dual guide nucleic acid comprises a crRNA-like ("CRISPR RNA" or "targeter" or "crRNA" or "crRNA repeat") molecule and a corresponding tracrRNA-like ("trans-acting CRISPR RNA" or "activator" or "tracrRNA") molecule. A crRNA-like molecule (targeter) comprises both the targeting segment (single stranded) of the guide nucleic acid and a stretch ("duplex-forming segment") of nucleotides that forms one half of the dsRNA duplex of the protein-binding segment of the guide nucleic acid. A corresponding tracrRNA-like molecule (activator) comprises a stretch of nucleotides (duplex-forming segment) that forms the other half of the dsRNA duplex of the protein-binding segment of the guide nucleic acid. In other words, a stretch of nucleotides of a crRNA-like molecule are complementary to and hybridize with a stretch of nucleotides of a tracrRNA-like molecule to form the dsRNA duplex of the protein-binding domain of the guide nucleic acid. As such, each crRNA-like molecule can be said to have a corresponding tracrRNA-like molecule. The crRNA-like molecule additionally provides the single stranded targeting segment. Thus, a crRNA-like and a tracrRNA-like molecule (as a corresponding pair) hybridize to form a dual guide nucleic acid. The exact sequence of a given crRNA or tracrRNA molecule is characteristic of the species in which the RNA molecules are found. A dual guide nucleic acid can include any corresponding activator and targeter pair.

The term "activator" is used herein to refer to a tracrRNA-like molecule of a dual guide nucleic acid (and of a single guide nucleic acid when the "activator" and the "targeter" are linked together by intervening nucleic acids). The term "targeter" is used herein to refer to a crRNA-like molecule of a dual guide nucleic acid (and of a single guide nucleic acid when the "activator" and the "targeter" are linked together by intervening nucleic acids). The term "duplex-forming segment" is used herein to mean the stretch of nucleotides of an activator or a targeter that contributes to the formation of the dsRNA duplex by hybridizing to a stretch of nucleotides of a corresponding activator or targeter molecule. In other words, an activator comprises a duplex-forming segment that is complementary to the duplex-forming segment of the corresponding targeter. As such, an activator comprises a duplex-forming segment while a targeter comprises both a duplex-forming segment and the targeting segment of the guide nucleic acid. A subject single guide nucleic acid can comprise an "activator" and a "targeter" where the "activator" and the "targeter" are covalently linked (e.g., by intervening nucleotides). Therefore, a dual guide nucleic acid can be comprised of any corresponding activator and targeted pair.

In a preferred embodiment the gene editing nuclei acid is bound to the gene editing nuclease to form a ribonucleoprotein gene editing complex comprising a gene editing nuclease covalently or non-covalently bound to an RNA.

In some particular embodiments of the invention the viral particle may further include a template nucleic acid (often referred to as swap nucleic acid). In some preferred embodiments the template nucleic acid has a 5' and 3' flanking region sequences homologous to sequences flanking the target sequence recognized by one or more gRNAs, and where preferably the template sequence is intended to be introduced. Such template nucleic acids may be provided as separate genetic constructs for example. Using gene editing nucleases a targeted double strand break can be introduced into a target genome and if a template nucleic acid exists having homology to the target site, the cellular repair mechanism will use the template nucleic acid to repair the introduced double strand break by homologous recombination and thereby introduce the template nucleic acid sequence into the target site. In this way genes can be introduced into a target genome, or even specific single nucleotide mutations. Such sequences used as template nucleic acids for homologous recombination are also referred to as donor nucleic acids. In some preferred embodiments of the invention the template nucleic acid is provided as a viral genome, preferably lentiviral genome, and has the structure of the two homology arms flanking a target genetic sequence to be introduced or to serve as donor nucleic acid for recombination, as mentioned, in context of a viral genome. In this case it is preferable that the viral genome comprising the template nucleic acid of the invention does not contain any viral genetic elements necessary for viral genomic integration or viral replication.

In an embodiment, the nucleic acid sequence that comprises or encodes the gRNA molecule sequence (e.g., targeted by the governing gRNA as described herein) further comprises a nucleic acid sequence that is capable of being used as a template nucleic acid, e.g., after being cleaved or excised (e.g., by the method described herein) from the nucleic acid sequence that comprises or encodes the gRNA molecule sequence, e.g., as a donor DNA for homologous recombination. The donor DNA may also be provided independently as a separate genetic construct from the gRNA. In an embodiment, a first governing gRNA molecule targets a region 5' of a nucleic acid sequence comprising the template nucleic acid sequence and a second governing gRNA molecule targets a region 3' of the nucleic acid sequence comprising the template nucleic acid sequence. For example, at least two (e.g., two, three, four, five or more) governing gRNAs can be used to produce one or more (e.g., two, three, four or more) template nucleic acids. In another embodiment, a single governing gRNA molecule targets both the regions 5' and 3' of the nucleic acid sequence comprising the template nucleic acid sequence. For example, the region (e.g., targeted by the governing gRNA molecule) 5' of the nucleic acid sequence comprising the template nucleic acid sequence can be the same or substantially the same as the region (e.g., targeted by the governing gRNAmolecule) 3' of the nucleic acid sequence comprising the template nucleic acid sequence. In an embodiment, the nucleic acid sequence comprising the template nucleic acid sequence is in a vector, e.g., a vector described herein. In an embodiment, the vector is a viral vector, e.g., an adeno-associated virus (AAV) vector.

In some preferred embodiments of the invention the term "cargo" shall denote any entity that is intended to be shuttled into a cell or cell-nucleus. Therefore, in some cases where the invention is concerned with gene editing approaches, the cargo compound is the shuttle compound itself. Hence, the invention relates to a viral particle wherein the gene editing nuclease protein is the cargo compound to be shuttled or delivered into a target cell or target cell nucleus. In other embodiments the cargo compound is a compound selected from a nucleic acid, a protein, a lipid, a small molecule, a macromolecule, a sugar, or combination thereof, and preferably the cargo compound is a component or a combination of components suitable for inducing a genome editing reaction in a target genome, such as a component or combination of components of the CRISPR/Cas9 genome editing system. Preferred examples of cargo compounds or gene editing targets to be delivered according to the invention into a cell or cell-nucleus, or to be genetically modified using the gene editing tools according to the invention, such as the gRNA and template nucleic acids described herein, are compounds and targets which were without the present invention "undruggable" in the sense of it was impossible to efficiently transfect or transduce them into, or modify or genetically alter the expression level within a target cell. Such compounds are selected from transcription factors (such as BET, bromodomain, β-Catenin, CBP, E2F1, HDM2, MDM2, NRF2, STAT3, TRPM2, c-MYC, eIF4e, KLF4, eya, NF-κB), telomerase proteins, ribonculeases, polo-like Kinasei, miRNAs (miR, microRNA), non-enzymatic proteins, scaffold proteins, regulatory proteins, loss of function targets (preferably proteins of the DNA repair machinery such as p53), RAS superfamily proteins, phosphatases (protein phosphatase A2, PP2Cdelta, PTP1B, SHP2, CDC25A/B, PRL, PTEN), trinucleotide repeats, splice factors / ribonucleoproteins.

In preferred embodiments the cargo compound is conjugated directly or indirectly to the shuttle compound. The shuttle compound is in this context the gene editing nuclease protein or a fragment or derivative thereof. The cargo compound may be directly conjugated to the shuttle compound by a covalent or non-covalent bond, and/or is indirectly conjugated via a linker, such as a peptide linker or a biotin-streptavidin linker. May possibilities are known to the skill artisan to design conjugate compounds or fusion proteins, and the invention shall not be restricted to a specific approach.

In some embodiments, in particular if genome editing is intended, the shuttle compound consists of the gene editing protein, or the fragment or derivative thereof.

The term "gene editing nuclease" in context of the present invention shall denote any enzyme suitable for the CRISPR gene editing, or any protein derivative therefrom, for example, mutated or inactivated versions of gene editing nucleases (as an example dCas9). In particular, gene editing nucleases are Cas9 proteins, or Cas9 related proteins. In the art to date, many alternative Cas9 nucleases, from many different species have been identified, as well as engineered for many different purposes. All such proteins shall be encompassed by the present invention. For example many Cas9 approaches and enzyme variants are reviewed in Nakede S et al, which is incorporated in its entirety (in particular table 1). As an additional example, Tsai et al. have designed recombinant dCas9-FoKI dimeric nucleases (RFNs) that can recognize extended sequences and edit endogenous genes with high efficiency in human cells. These nucleases comprise a dimerization-dependent wild type Fokl nuclease domain fused to a catalytically inactive Cas9 (dCas9) protein. Dimers of the fusion proteins mediate sequence specific DNA cleavage when bound to target sites composed of two half-sites (each bound to a dCas9 (i.e., a Cas9 nuclease devoid of nuclease activity) monomer domain) with a spacer sequence between them. The dCas9-FoKI dimeric nucleases require dimerization for efficient genome editing activity and thus, use two gRNAs for introducing a cut into DNA. The recombinant gene editing nuclease protein that may be used in accordance with the present invention is i) derived from a naturally occurring Cas; and ii) has (or originally had) a nuclease (or nickase) activity to introduce a DSB (or two SSBs in the case of a nickase) in cellular, and in particular genomic, DNA when in the presence of appropriate gRNA(s). Thus, as used herein, the term "gene editing nuclease" refers to a recombinant protein which is derived from a naturally occurring Cas nuclease which has (or had) nuclease or nickase activity and which functions with a gRNA/gDNA to introduce DSBs (or one or two SSBs) in the targets of interest. In embodiments, the gene editing nuclease protein is spCas9. In embodiments, the gene editing nuclease is Cpfi. In another embodiment, the gene editing nuclease protein is a Cas9 protein having a nickase activity. As used herein, the term "Cas9 nickase" refers to a recombinant protein which is derived from a naturally occurring Cas9 and which has one of the two nuclease domains inactivated such that it introduces single stranded breaks (SSB) into the DNA. It can be either the RuvC or HNH domain. In a further embodiment, the Cas protein is a dCas9 protein fused with a dimerization-dependant FoKI nuclease domain. Exemplary gene editing nuclease fusion proteins that may be used in accordance with the present invention are provided in the following list of enzymes^{[10]}: CtIP, RecA, Fok1, Cytidine Deaminases like APOBEC1, APOBEC3A, pmCDA1, , Adenine Deaminases like Cry2, Cibi, cibn, abi-pyl1, gidi-gai, ERT2 FKBP-FRB, PHYB-PIF, CRY2PHR-CIBN, FKF1-GI, Vpr, P65, Vp64, Rta, Sam, sunTAG, prdm9, sid4x, dot1l, teti, Krab, Dnmt3a, MQ3 methylase, CpG Mtlase, LSDi, P300, ZFP, TALE, DHFR, ER50, N-DHFRCas9-C-DHFR, GFP / RFP / BFP , intein 37R3-2, pdDronpa, RsLOV, , K866, VQR EQR VRER or derivatives of the above mentioned CRISPR/Cas9-fusion proteins like APOBEC-Cas9-UGI (BE3), BE4max, ABEmax, AncBE4max, Target-AID, TAM, CRISPR-X YE1-BE3, EE-BE3, YE2-BE3, or YEE-BE3, Cas9 CTD and PACE derived xCas9. In general a variant of Cas9 can be a Cas9 nuclease that is obtained by protein engineering or by random mutagenesis (i.e., is non-naturally occurring). Such Cas9 variants remain functional and may be obtained by mutations (deletions, insertions and/or substitutions) of the amino acid sequence of a naturally occurring Cas9, such as that of *S. pyogenes* (which is preferred). An example of a Cas9 protein which is preferred is the Cas9 used in the examples^{[11]}. An original Cas9 amino acid sequence is provided SEQ ID NO: 1, which may be preferred in some instances or modified according to ^{[11]}. Another codon optimized Cas9 sequence suitable for the herein disclosed invention is shown in SEQ ID NO: 2. Cas9 proteins or polypeptides of the invention are in some embodiments polypeptides having at least 50%, 60%, 70%, 80%, 90%, 95%, 96%, 97% 98, 99%, or 100% sequence identity to the amino acid sequence of SEQ ID NO: 1 or 2.

As used herein, the terms "identical" or percent "identity", when used anywhere herein in the context of two or more nucleic acid or protein/polypeptide sequences, refer to two or more sequences or sub-sequences that are the same or have (or have at least) a specified percentage of amino acid residues or nucleotides that are the same (i.e., at, or at least, about 60% identity, preferably at, or at least, 65%, 70%, 75%, 80%, 85%, 90%, 91%, 92%, 93% or 94%, identity, and more preferably at, or at least, about 95%, 96%, 97%, 98%, 99%, or higher identity over a specified region - preferably over their full length sequences - , when compared and aligned for maximum correspondence over the comparison window or designated region) as measured using a sequence comparison algorithms, or by manual alignment and visual inspection (see, e.g., NCBI web site). In a particular embodiment, for example when comparing the protein or nucleic acid sequence of the gene editing nuclease protein of the invention to for example a reference (such as Cas9), the percentage identity can be determined by the Blast searches provided in NCBI; in particular for amino acid identity, those using BLASTP 2.2.28+ with the following parameters: Matrix: BLOSUM62; Gap Penalties: Existence: 11, Extension: 1; Neighboring words threshold: 11; Window for multiple hits: 40.

The viral particle of the invention may in other embodiments be provided in context of a recombinant cell expressing such a particle. Thence the present invention in additional aspects pertains to a recombinant cell, such a mammalian cell, comprising genetic constructs suitable for and sufficient for expression of the viral particle of the invention.

In **a second aspect,** the invention pertains to a method for producing a viral particle for use in the delivery of a cargo compound into a cell and/or into a cell nucleus, the method comprising the step of
(e) Introducing into a viral packaging cell one or more genetic constructs for the expression of a shuttle compound and a cargo compound, wherein the one or more genetic constructs comprise (i) a first expressible sequence encoding a gene-editing nuclease protein, or a derivative or fragment thereof, and (ii) a second expressible sequence encoding a cargo compound; alternatively, wherein (i) is not introduced into the viral packaging cell but provided as a stable, preferably genome integrated, expression construct in said viral packaging cell;
(f) Expressing the first and the second expressible sequences in the viral packaging cell,
(g) Expressing viral particle components for viral packaging in the viral packaging cell and thereby packaging the shuttle compound and the cargo compound into the viral particle, and
(h) Harvesting the so produced viral particle.

In some preferred embodiments of the invention, the cargo compound and the shuttle compound are directly or indirectly conjugated to each other in the packaging cell before being packaged into the viral particle.

Particular embodiments described for third aspect are also preferred for the second aspect. However, generally, the present disclosure shall encompass all combinations or aspects and embodiments where they make technical sense in order provide a concise description without extensive redundancies and repetition.

In **a third aspect,** the invention pertains to a method for producing a viral particle for use in the delivery of gene-editing components into a target cell and/or into a target cell nucleus, the method comprising the step of
(e) Introducing into a viral packaging cell one or more genetic constructs for the expression of gene-editing components, wherein the one or more genetic constructs comprise (i) a first expressible sequence encoding a gene-editing nuclease, or a derivative or fragment thereof and (ii) a second expressible sequence encoding at least for one gene-editing guide nucleic acid; alternatively, wherein (i) is not introduced into the viral packaging cell but provided as a stable, preferably genome integrated, expression construct in said viral packaging cell;
(f) Expressing the first and the second expressible sequences in the viral packaging cell,
(g) Expressing viral particle components for viral packaging in the viral packaging cell, and
(h) Harvesting the so produced viral particle.

As used herein, the term "packaging cell" refers to a cell which contains those elements necessary for production of infectious recombinant virus which are lacking in the viral genome provided by viral nucleic acid transgene construct. Such packaging cells are capable of expressing viral structural proteins (such as gag-pol and env, which may be codon optimised) but they do not contain a packaging signal. The term "packaging signal" which is referred to interchangeably as "packaging sequence" or "psi" is used in reference to the non-coding, cis-acting sequence required for encapsidation of retroviral RNA strands during viral particle formation.

In one further embodiment of the invention the method may comprise a step of Introducing into the viral packaging cell (for example in step (a)) one or more genetic constructs comprising a sequence that serves as a template nucleic acid as described herein before. In such instances one, or two or more, guide nucleic acids may be introduced into the packaging cell.

The packaging cells of the present invention may be any suitable cell type. Packaging cells are generally mammalian cells but can be, for example, insect cells. By using packaging cell lines of the present invention, it is possible to propagate and isolate quantities of viral particles of the invention (e.g. to prepare suitable titres of the viral particles) for subsequent transduction of a site of interest. The packaging cell lines are useful for providing the gene products necessary to encapsidate and provide a membrane protein for a high titre vector particle production. The packaging cell may be a cell cultured in vitro such as a tissue culture cell line. The packaging cell of the present invention may be, but is not limited to, a mammalian cell such as a murine fibroblast derived cell or a human cell line. The packaging cell or producer cell of the present invention may be a human cell line, such as for example: HEK293, HEK293T, HEK293FT, BHK21, BSR-T7/5, TE671, HT1080, 3T3, or K562.

A packaging cell of the invention preferably stably or transiently expresses Gag, Pol and Env proteins and potentially Cas9 and T7 RNA polymerase. The viral packaging cell may be a cell which comprises, and/or was transfected with, a viral packaging plasmid such as pMD2.G. as used herein. Such constructs may be stably integrated into the packaging cells. The term "stably integrated" means that the foreign genes become integrated into the cell's genome. The packaging cell line produces the proteins required for packaging retroviral RNA but it importantly lacks the ability to encapsidate viral genomic nucleic acids and for example due to the lack of a psi region.

Preferably the one or more genetic constructs and the packaging cell do not comprise a functional integrase, preferably viral integrase, more preferably lentiviral integrase. By providing viral particles that do not have the ability to uncontrollably integrate into a host genome, the present invention provides safe and efficient shuttles for gene editing. Hence, in preferred embodiments of the invention the one or more genetic constructs, when combined, do not comprise all viral components necessary for viral integration into a target host genome, preferably wherein the one or more genetic constructs do not comprise a functional long terminal repeat (LTR) structure, and/or do not comprise an expressible sequence of a functional integrase protein.

In context of the invention the expressible sequence encoding at least for one gene-editing guide nucleic acid is under control of an RNA polymerase promoter, preferably an U6, U7, H1 or T7 promoter. Expression systems for guide nucleic acids are well known and not restricted in their use for the present invention in any way.

In other or additional preferred embodiments the expressible sequence encoding a gene-editing nuclease is under control of eukaryotic promotors like the elongation factor 1-alpha (EF1a, the Ubiquitin C (UbiC), the phosphoglycerate kinase (PGK) or the spleen focus-forming virus (SFFV) promoter.

It shall be understood that the first and the second expressible sequences may be located on one genetic construct, preferably wherein the genetic construct is a lentiviral-derived vector such as a pLentiCRISPRv2.

In **a fourth aspect,** the invention pertains to a viral particle produced with a method according to the second or third aspect of the invention.

In **a fifth aspect,** the invention pertains to a composition comprising a viral particle according to the first or fourth aspect of the invention, together with a carrier and/or excipient. Alternatively such compositions may comprise a recombinant cell expressing such inventive particles, or comprising all genetic constructs suitable for the expression of the inventive viral particles.

The composition of the invention is preferably suitable for the transduction of an animal cell, such as a human cell. Hence, the composition in some additional and alternative embodiments are suitable or use in gene therapy of a subject.

The composition which is suitable for use in medicine is preferably a pharmaceutical composition, and a composition wherein the carrier and/or excipient are pharmaceutically acceptable, preferably in a form suitable for administration of the composition to a subject.

The present invention further includes various pharmaceutical compositions comprising polynucleotides, vectors, and polypeptides contemplated herein and a pharmaceutically acceptable carrier and/or excipient. Most preferably the pharmaceutical compositions of the invention comprise a viral particle of the invention as described before. As used herein "pharmaceutically acceptable carrier" includes any and all solvents, dispersion media, coatings, antibacterial and antifungal agents, isotonic and absorption delaying agents, and the like that are physiologically compatible, including pharmaceutically acceptable cell culture media. Pharmaceutically acceptable carriers include sterile aqueous solutions, dispersions or aerosols and sterile powders for the extemporaneous preparation of sterile injectable solutions or dispersion. The use of such media and agents for pharmaceutically active substances is well known in the art. Except insofar as any conventional media or agent is incompatible with the vectors contemplated herein, use thereof in the pharmaceutical compositions of the invention is also contemplated.

The compositions of the invention may comprise one or more polypeptides, polynucleotides, and vectors comprising same, transduced cells, etc., as described herein, formulated in pharmaceutically-acceptable or physiologically-acceptable solutions for administration to a cell or an animal, either alone, or in combination with one or more other modalities of therapy. It will also be understood that, if desired, the compositions of the invention may be administered in combination with other agents as well, such as, e.g., cytokines, e.g., anti-inflammatory cytokines, growth factors, hormones, small molecules or various pharmaceutically-active agents. There is virtually no limit to other components that may also be included in the compositions, provided that the additional agents do not adversely affect the ability of the composition to deliver the intended (gene) therapy.

In the pharmaceutical compositions contemplated herein, formulation of pharmaceutically-acceptable excipients and carrier solutions is well-known to those of skill in the art, as is the development of suitable dosing and treatment regimens for using the particular compositions described herein in a variety of treatment regimens, including e.g., oral, parenteral, intravenous, intranasal, intramuscular, intrathecal, intraneural, intraganglion, intracranial, and intraventricular administration and formulation.

In certain circumstances it will be desirable to deliver the compositions disclosed herein parenterally, intravenously, intramuscularly, intraperitoneally, intrathecally, intraneurally, intraganglionicly, intracranially, or intraventricularly. Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms.

The compositions disclosed herein may be formulated in a neutral or salt form. Pharmaceutically-acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective. The formulations are easily administered in a variety of dosage forms such as injectable solutions, drug-release capsules, and the like.

As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The phrase "pharmaceutically-acceptable" refers to molecular entities and compositions that do not produce an allergic or similar untoward reaction when administered to a human. The preparation of an aqueous composition that contains a protein as an active ingredient is well understood in the art. Typically, such compositions are prepared as injectables, either as liquid solutions or suspensions; solid forms suitable for solution in, or suspension in, liquid prior to injection can also be prepared. The preparation can also be emulsified.

In particular, the compositions of the present invention may be formulated for delivery either encapsulated in a lipid particle, a liposome, an exosome, a vesicle, a nanosphere, a nanoparticle or the like, insofar they can encase a viral particle of the invention such as a capsid. The formulation and use of such delivery vehicles can be carried out using known and conventional techniques. The formulations and compositions of the invention may comprise one or more polypeptides, polynucleotides, and small molecules, as described herein, formulated in pharmaceutically-acceptable or physiologically-acceptable solutions (e.g., culture medium) for administration to a cell or an animal, either alone, or in combination with one or more other modalities of therapy. It will also be understood that, if desired, the compositions of the invention may be administered in combination with other agents as well, such as, e.g., cells, other proteins or polypeptides or various pharmaceutically-active agents.

In a particular embodiment, a formulation or composition according to the present invention comprises a cell contacted with a combination of any number of polypeptides, polynucleotides, and viral particles, as contemplated herein.

In certain aspects, the present invention provides formulations or compositions suitable for the delivery of viral particles

In certain aspects, the present invention provides pharmaceutically acceptable compositions which comprise a therapeutically-effective amount of one or more viral particles of the invention as described herein, formulated together with one or more pharmaceutically acceptable carriers (additives) and/or diluents (e.g., pharmaceutically acceptable cell culture medium).

Particular embodiments of the invention may comprise other formulations, such as those that are well known in the pharmaceutical art, and are described, for example, in Remington: The Science and Practice of Pharmacy, 20th Edition. Baltimore, Md.: Lippincott Williams & Wilkins, 2000.

In **a sixth aspect,** the invention pertains a product for use in the treatment of a disease, wherein the product is a viral particle according to the first or fourth aspect of the invention, or is a composition according to the fifth aspect of the invention.

Preferably the viral particle of the invention, or a recombinant cell expressing such a particle, is preferably administered to a subject in need of the treatment in an amount that is therapeutically effective. The viral particle of the invention, may be administered preferably at a dose range between 1 x 10¹⁰ viral particles (vp)/kg and 1 x 10¹⁵ vp/kg. A dose range between 1x10¹¹ and 1x10¹⁵ vp/kg is more likely to be effective in humans because these doses are expected to result in large transduction efficiency of a target tissue.

In **a seventh aspect,** the invention pertains an *in vitro* use of (i) a viral particle according to the first or fourth aspect of the invention, or (ii) of a composition according to the fifth aspect of the invention, in the targeted delivery of one or more cargo compounds into a target cell and/or into a target cell nucleus.

In **an eighth aspect,** the invention pertains to a method of editing a genomic sequence in a target cell, comprising a step of contacting (or transducing) the target cell and/or a target cell nucleus, with a viral particle according to the first or fourth aspect of the invention, or with a composition according to the fifth aspect of the invention.

In **a ninth aspect,** the invention pertains to a system for editing a target genome, the system comprising
(iii) a genetic expression construct comprising an expressible sequence of a gene-editing nuclease, or a fragment or derivative thereof,
(iv) genetic expression construct comprising an expressible expression cassette for introducing a sequence of a gene-editing guide nucleic acid sequence,
wherein the genetic construct of (i) an (ii) can be provided on one nucleic acid molecule or separate nucleic acid molecules, and wherein the genetic construct(s) is(are) not able to integrate into a target genome.

In preferred embodiments the system of the invention further comprises a viral packaging cell or a plasmid encoding components for viral packaging, preferably encoding for gag, pol and env proteins. Viral packaging cells are described in detail herein above.

The system of the invention may further include a third nucleic acid construct comprising a template sequence as described herein above.

The system in particular embodiments comprises the genetic constructs which further comprise a promoter sequence operably linked to said expressible sequence, or expressible expression cassette.

In **a tenth aspect,** the invention pertains to a kit of parts comprising one or more, or all of, the components of the system according to the ninth aspect of the invention.

In view of the above, it will be appreciated that the present invention also relates to the following itemised embodiments:
Item 1: A viral particle for delivering cargo into a cell and/or into a cell nucleus, the viral particle comprising a first particle coat encasing a first particle volume, wherein the first particle volume comprises the cargo compound, and a shuttle compound, the shuttle compound comprising a gene-editing nuclease protein, or a fragment or derivative thereof.
Item 2: The viral particle according to item 1, wherein the cargo compound is the shuttle compound, or wherein the cargo compound is selected from a nucleic acid, a protein, a lipid, a small molecule, a macromolecule, a sugar, or combination thereof, and preferably the cargo compound is a component or a combination of components suitable for inducing a genome editing reaction in a target genome, such as a component or combination of components of the CRISPR/Cas9 genome editing system.
Item 3: The viral particle according to item 1 or 2, wherein the first particle coat is a viral capsid.
Item 4: The viral particle according to any one of items 1 to 3, wherein the first particle coat does not comprise a lipid mono- or bilayer, preferably wherein the viral particle is not an exosome, liposome, micelle or other particle essentially composed of amphiphilic lipids.
Item 5: The viral particle according to any one of items 1 to 4, wherein the gene-editing nuclease protein is essentially absent from the first particle coat.
Item 6: The viral particle according to item 1 to 5, wherein the gene editing nuclease protein is not fused to a structural component of the first particle coat, preferably wherein such structural component is a protein, such as a viral coat protein.
Item 7: The viral particle according to any one of items 1 to 6, essentially not comprising a functional integrase, preferably essentially not comprising a functional integrase protein and essentially not comprising an expressible and/or translatable nucleic acid sequence encoding for a functional integrase protein.
Item 8: The viral particle according to any one of items 1 to 7, essentially not comprising a full or partial viral genome.
Item 9: The viral particle according to any one of items 1 to 8, wherein the first particle volume further comprises at least one species of a gene-editing guide nucleic acid, such as a guide RNA (gRNA), or single guide RNA (sgRNA), or a guide DNA (gDNA) or single guide DNA (sgDNA) or combinations or variants thereof.
Item 10: The viral particle according to any one of items 1 to 9, further comprising at least one species of template nucleic acid, preferably having a 5' and 3' flanking region sequences homologous to sequences flanking the target sequence recognized by one or more gRNAs, and where preferably the template sequence is intended to be introduced.
Item 11: The viral particle according to item 9 or 10, wherein the gRNA is bound to the gene editing nuclease to form a ribonucleoprotein gene editing complex comprising a gene editing nuclease covalently or non-covalently bound to an RNA.
Item 12: The viral particle according to any one of items 1 to 11, further comprising a second particle coat encasing the first particle coat within an intermediate particle volume.
Item 13: The viral particle according to item 12, wherein the second particle coat is composed of a lipid bilayer, and wherein the first particle coat forms a viral capsid.
Item 14: The viral particle according to any one of items 1 to 13, wherein the viral particle is a retrovirus derived particle, preferably a lentivirus derived particle.
Item 15: The viral particle according to any one of items 1 to 14, wherein the gene-editing nuclease protein is a protein selected from Cas9, or a variant thereof such as dCas9, or Cpfi, or a variants or derivatives thereof, or is a fusion protein thereof with a fusion partner selected from the group CtIP, RecA, Fok1, Cytidine Deaminases like APOBEC1, APOBEC3A, pmCDA1,, Adenine Deaminases like Cry2, Cibi, cibn, abi-pyl1, gidi-gai, ERT2 FKBP-FRB, PHYB-PIF, CRY2PHR-CIBN, FKF1-GI, Vpr , P65, Vp64, Rta, Sam, sunTAG, prdm9, sid4x, dot1l, teti, Krab, Dnmt3a, MQ3 methylase, CpG Mtlase, LSDi, P300, ZFP, TALE, DHFR, ER50, N-DHFRCas9-C-DHFR, GFP / RFP / BFP, intein 37R3-2, pdDronpa, RsLOV, , K866, VQR EQR VRER or derivatives of the above mentioned CRISPR/Cas9-fusion proteins like APOBEC-Cas9-UGI (BE3), BE4max, ABEmax, AncBE4max, Target-AID , TAM, CRISPR-X YE1-BE3, EE-BE3, YE2-BE3, or YEE-BE3, Cas9 CTD and PACE derived xCas9.
Item 16: The viral particle according to any one of items 1 to 15, wherein the first particle coat, or a structural component of the first particle coat, does not comprise, or does not contain, or is not covalently fused to, a gene-editing nuclease protein.
Item 17: The viral particle according to any one of items 1 to 16, wherein the particle comprises a particle surface-presented targeting molecule suitable for targeting the delivery of the particle to a target cell type.
Item 18: The viral particle according to item 17, wherein the target cell type is a fetal cord blood cell, bone marrow cells, hematopoietic progenitor cells, or any immunological cell, such as antigen presenting cells (dendritic cells), natural killer cells, lymphocytes, T cells, B cells, macrophages and medullary thymic epithelial cells (mTEC), myeloid derived suppressor cells, T-regulatory cells, a fibroblast cell, a brain cell, a lung cell, a liver cell, a muscle cell, a pancreatic cell, an endothelial cell, a cardiac cell, a skin cell, a bone marrow stromal cell, and an eye cells, a pancreatic ductal cell, a neural precursor, or a mesodermal stem cell. Stem or progenitor cell, hepatic sinusoidal endothelium cell.
Item 19: The viral particle according to item 17 and 18, wherein the particle surface-presented targeting molecule is a protein, such as an antibody-like proteins or DARPins.
Item 20: The viral particle according to any one of items 1 to 19, wherein the fragment or derivative of the gene-editing nuclease protein retains the ability of the gene-editing nuclease protein to be packaged into a viral capsid.
Item 21: The viral particle according to any one of items 1 to 20, wherein the cargo compound is conjugated directly or indirectly to the shuttle compound.
Item 22: The viral particle according to item 21, wherein the cargo compound is directly conjugated to the shuttle compound by a covalent or non-covalent bond, and/or is indirectly conjugated via a linker, such as a peptide linker or a biotin-streptavidin linker.
Item 23: The viral particle according to any one of items 1 to 22, wherein the shuttle compound consists of the gene editing protein, or the fragment or derivative thereof.
Item 24: A method for producing a viral particle for use in the delivery of a cargo compound into a cell and/or into a cell nucleus, the method comprising the step of a) Introducing into a viral packaging cell one or more genetic constructs for the expression of a shuttle compound and a cargo compound, wherein the one or more genetic constructs comprise (i) a first expressible sequence encoding a gene-editing nuclease protein, or a derivative or fragment thereof, and (ii) a second expressible sequence encoding a cargo compound; alternatively, wherein (i) is not introduced into the viral packaging cell but provided as a stable, preferably genome integrated, expression construct in said viral packaging cell, b) Expressing the first and the second expressible sequences in the viral packaging cell, c) Expressing viral particle components for viral packaging in the viral packaging cell and thereby packaging the shuttle compound and the cargo compound into the viral particle, d)Harvesting the so produced viral particle.
Item 25: The method according to item 24, wherein the cargo compound and the shuttle compound are directly or indirectly conjugated to each other in the packaging cell before being packaged into the viral particle.
Item 26: A method for producing a viral particle for use in the delivery of gene-editing components into a target cell and/or into a target cell nucleus, the method comprising the step of a) Introducing into a viral packaging cell one or more genetic constructs for the expression of gene-editing components, wherein the one or more genetic constructs comprise (i) a first expressible sequence encoding a gene-editing nuclease, or a derivative or fragment thereof and (ii) a second expressible sequence encoding at least for one gene-editing guide nucleic acid; alternatively, wherein (i) is not introduced into the viral packaging cell but provided as a stable, preferably genome integrated, expression construct in said viral packaging cell; b) Expressing the first and the second expressible sequences in the viral packaging cell, c) Expressing viral particle components for viral packaging in the viral packaging cell, d) Harvesting the so produced viral particle.
Item 27: The method according to any one of items 24 to 26, wherein the one or more genetic constructs and the packaging cell do not comprise a functional integrase, preferably viral integrase, more preferably lentiviral integrase.
Item 28: The method according to any one of items 24 to 27, wherein the one or more genetic constructs, when combined, do not comprise all viral components necessary for viral integration into a target host genome, preferably wherein the one or more genetic constructs do not comprise a functional long terminal repeat (LTR) structure, and/or do not comprise an expressible sequence of a functional integrase protein.
Item 29: The method according to any one of items 26, and 27 or 28, when referring to item 26, wherein the expressible sequence encoding at least for one gene-editing guide nucleic acid is under control of an RNA polymerase promoter, preferably an U6, U7, H1 or T7 promoter.
Item 30: The method according to any one of items 24 to 29, wherein the packaging cell is a cell stably expressing CRISPR/Cas9 protein and T7 RNA polymerase. (which increases efficiency of sgRNA loading of Cas9).
Item 31: The method according to any one of items 24 to 30, wherein the expressible sequence encoding a gene-editing nuclease is under control of eukaryotic promotors like the elongation factor 1-alpha (EF1a, the Ubiquitin C (UbiC), the phosphoglycerate kinase (PGK) or the spleen focus-forming virus (SFFV) promoter.
Item 32: The method according to any one of items 24 to 31, wherein the first and the second expressible sequences are located on one genetic construct, preferably wherein the genetic construct is a lentiviral-derived vector such as a pLentiCRISPRv2.
Item 33: The method according to any one of items 24 to 32, wherein the viral particle components, such as viral packaging proteins, are expressed from viral gag, pol and env genes comprised within the viral packaging cell.
Item 34: The method according to any one of items 24 to 33, wherein the viral packaging cell is a cell which comprises, and/or was transfected with, viral packaging plasmids such as pMD2.G and psPax2.
Item 35: The method according to any one of items 24 to 34, wherein the method is suitable for producing a viral particle according to any one of items 1 to 23, preferably wherein the viral particle is the viral particle according to any one of items 1 to 23.
Item 36: A viral particle produced with a method according to any one of items 24 to 35.
Item 37: A composition comprising a viral particle according to any one of items 1 to 23 and 36, together with a carrier and/or excipient.
Item 38: The composition according to item 37, suitable for the transduction of an animal cell, such as a human cell.
Item 39: The composition according to item 37 or 38, which is a pharmaceutical composition, and wherein the carrier and/or excipient are pharmaceutically acceptable.
Item 40: The composition according to any one of items 37 to 39, in a form suitable for administration of the composition to a subject.
Item 41: A product for use in the treatment of a disease, wherein the product is a viral particle according to any one of items 1 to 23, or 36, or is a composition according to any one of items 37 to 40.
Item 42: An in vitro use of (i) a viral particle according to any one of items 1 to 23, and 36, or (ii) of a composition according to any one of items 37 to 40, in the targeted delivery of one or more cargo compounds into a target cell and/or into a target cell nucleus.
Item 43: A method of editing a genomic sequence in a target cell, comprising a step of contacting (or transducing) the target cell and/or a target cell nucleus, with a viral particle according to any one of items 1 to 23, and 36, or with a composition according to any one of items 37 to 40.
Item 44: A system for editing a target genome, the system comprising (i) A genetic expression construct comprising an expressible sequence of a gene-editing nuclease, or a fragment or derivative thereof, (ii) A genetic expression construct comprising an expressible expression cassette for introducing a sequence of a gene-editing guide nucleic acid sequence, wherein the genetic construct of (i) an (ii) can be provided on one nucleic acid molecule or separate nucleic acid molecules, and wherein the genetic construct(s) is(are) not able to integrate into a target genome.
Item 45: The system according to item 44, further comprising a viral packaging cell or a plasmid encoding components for viral packaging, preferably encoding for gag, pol and env proteins.
Item 46: The system according to item 44 or 45, wherein the genetic constructs further comprise a promoter sequence operably linked to said expressible sequence, or expressible expression cassette.
Item 47: A kit of parts comprising one or more, or all of, the components of the system according to any one of items 44 to 46.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Embodiments defined by terms such as "essentially", "essentially not" or "comprising essentially" or "consisting essentially of' pertain to such embodiments where in the majority such feature is present (above 95%, preferably above 99% or even more such as 99,9%). For example with respect of compositions a small amount of impurities might remain whereas the composition is "essentially" pure. In some preferred embodiments such embodiments may be completely and not essentially comprising such referred feature.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:
**Figure 1**: shows protein expression of CRISPR/Cas9 from Lentivirus like particles normalized to p24 gag viral capsid protein.
**Figure 2**: shows protein expression of flag-tagged CRISPR/Cas9 normalized to GAPDH from HEK/293 eGFP+ reporter cells transduced with either integrating lentivirus coding for CRISPR/Cas9 as well as sgRNA or lentiviral particles containing Cas9 Protein and lentiviral genome coding for the sgRNA with the indicated modifications. Protein expression was analysed 12 hours post transduction and 10 days post transduction.
**Figure 3****:** shows the generation of a plasmid to co-express sgRNA under control of U6 promoter and CRISPR/Cas9 under control of eukaryotic promotors like SFFV, EFia, UbiC or PGK for generation of CaPRiCoRN System (CaPRiCoRN is an acronym for **Ca**s**9 Ri**bonucleoprotein**C**ontaining**R**etroviral**N**anoparticles).
**Figure 4**: shows protein expression of CRISPR/Cas9 from Lentivirus like particles normalized to p24 gag viral capsid protein.
**Figure 5**: shows protein expression of p24 gag viral capsid protein in cytoplasmic, organelle and nuclear fractions.
**Figure 6****:** shows efficiency of gene disruption in HEK293 eGFP+ reporter cells transduced with CaPRiCoRN System analysed by flow cytometry. Depicted results are representative of n=3 independent experiments.
**Figure 7**: shows a sketch of the generation and delivery pathway of Cas9/gRNA ribonuleoprotein into a target cell nucleus according to the invention.
**Figure 8****:** shows a sketch of the delivery of the invention including lentiviral genome as a template for precise homology-directed repair.

And the sequences show:
**SEQ ID NO: 1** - CRISPR-associated endonuclease Cas9/Csn1 *Streptococcus pyogenes* serotype M1 
10 20 30 40 50

60 70 80 90 100

110 120 130 140 150

160 170 180 190 200

210 220 230 240 250

260 270 280 290 300

310 320 330 340 350

360 370 380 390 400

410 420 430 440 450

460 470 480 490 500

510 520 530 540 550

560 570 580 590 600

610 620 630 640 650

660 670 680 690 700

710 720 730 740 750

760 770 780 790 800

810 820 830 840 850

860 870 880 890 900

910 920 930 940 950

960 970 980 990 1000

1010 1020 1030 1040 1050

1060 1070 1080 1090 1100
 

      1110       1120       1130       1140       1150

      1160       1170       1180       1190       1200

      1210       1220       1230       1240       1250

      1260       1270       1280       1290       1300

      1310       1320       1330       1340       1350

      1360

**SEQ ID NO: 2** human codon optimized CRISPR-associated endonuclease Cas9/Csn1 *Streptococcus pyogenes with dual nuclear localization sequence and C-terminal tag*

MYPYDVPDYASPKKKRKVEASDKKYSIGLDIGTNSVGWAVITDEYKVPSKKFKVLGNTDRHSIKKNLIG
ALLFDSGETAEATRLKRTARRRYTRRKNRICYLQEIFSNEMAKVDDSFFHRLEESFLVEEDKKHERHPI
FGNIVDEVAYHEKYPTIYHLRKKLVDSTDKADLRLIYLALAHMIKFRGHFLIEGDLNPDNSDVDKLFIQ
LVQTYNQLFEENPINASGVDAKAILSARLSKSRRLENLIAQLPGEKKNGLFGNLIALSLGLTPNFKSNF
DLAEDAKLQLSKDTYDDDLDNLLAQIGDQYADLFLAAKNLSDAILLSDILRVNTEITKAPLSASMIKRY
DEHHQDLTLLKALVRQQLPEKYKEIFFDQSKNGYAGYIDGGASQEEFYKFIKPILEKMDGTEELLVKLN
REDLLRKQRTFDNGSIPHQIHLGELHAILRRQEDFYPFLKDNREKIEKILTFRIPYYVGPLARGNSRFA
WMTRKSEETITPWNFEEVVDKGASAQSFIERMTNFDKNLPNEKVLPKHSLLYEYFTVYNELTKVKYVTE
GMRKPAFLSGEQKKAIVDLLFKTNRKVTVKQLKEDYFKKIECFDSVEISGVEDRFNASLGTYHDLLKII
KDKDFLDNEENEDILEDIVLTLTLFEDREMIEERLKTYAHLFDDKVMKQLKRRRYTGWGRLSRKLINGI
RDKQSGKTILDFLKSDGFANRNFMQLIHDDSLTFKEDIQKAQVSGQGDSLHEHIANLAGSPAIKKGILQ
TVKVVDELVKVMGRHKPENIVIEMARENQTTQKGQKNSRERMKRIEEGIKELGSQILKEHPVENTQLQN
EKLYLYYLQNGRDMYVDQELDINRLSDYDVDHIVPQSFLKDDSIDNKVLTRSDKNRGKSDNVPSEEVVK
KMKNYWRQLLNAKLITQRKFDNLTKAERGGLSELDKAGFIKRQLVETRQITKHVAQILDSRMNTKYDEN
DKLIREVKVITLKSKLVSDFRKDFQFYKVREINNYHHAHDAYLNAVVGTALIKKYPKLESEFVYGDYKV
YDVRKMIAKSEQEIGKATAKYFFYSNIMNFFKTEITLANGEIRKRPLIETNGETGEIVWDKGRDFATVR
KVLSMPQVNIVKKTEVQTGGFSKESILPKRNSDKLIARKKDWDPKKYGGFDSPTVAYSVLVVAKVEKGK
SKKLKSVKELLGITIMERSSFEKNPIDFLEAKGYKEVKKDLIIKLPKYSLFELENGRKRMLASAGELQK
GNELALPSKYVNFLYLASHYEKLKGSPEDNEQKQLFVEQHKHYLDEIIEQISEFSKRVILADANLDKVL
SAYNKHRDKPIREQAENIIHLFTLTNLGAPAAFKYFDTTIDRKRYTSTKEVLDATLIHQSITGLYETRI
DLSQLGGDKRPAATKKAGQAKKKKDYKDDDDKGS*

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:
**Example 1**: Lentivirus like particles allow for transient gene editing by viral-genomic-RNA-independent incorporation of functional CRISPR/Cas9 ribonucleoparticles by capsid mediated nuclear shuttle.

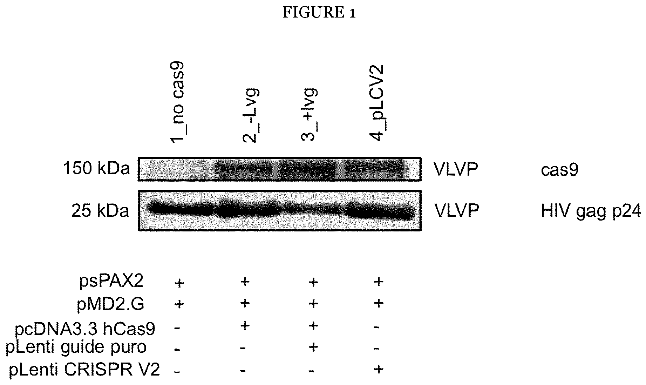

To address the issues of applying gene editing approaches efficiently in clinical practise, the inventors sought a method to combine the benefits of a time-limited application of CRISPR / Cas9 ribonucleoproteins with those of non-toxic biological administration via retroviral particles, without dispensing with broad therapeutic applicability of such a method to a variety of diseases. As a result of the observation that CRISPR / Cas9 protein can be detected in a regular generation of lentiviruses in virions, the inventors surprisingly found that the presence of CRISPR / Cas9 protein is not dependent on the presence of a lentiviral genome. (Figure 1). This circumstance allowed an adapterless incorporation of CRISPR / Cas9 protein into retroviral particles in the total absence of viral genetic information so that integration into a host genome is impossible.

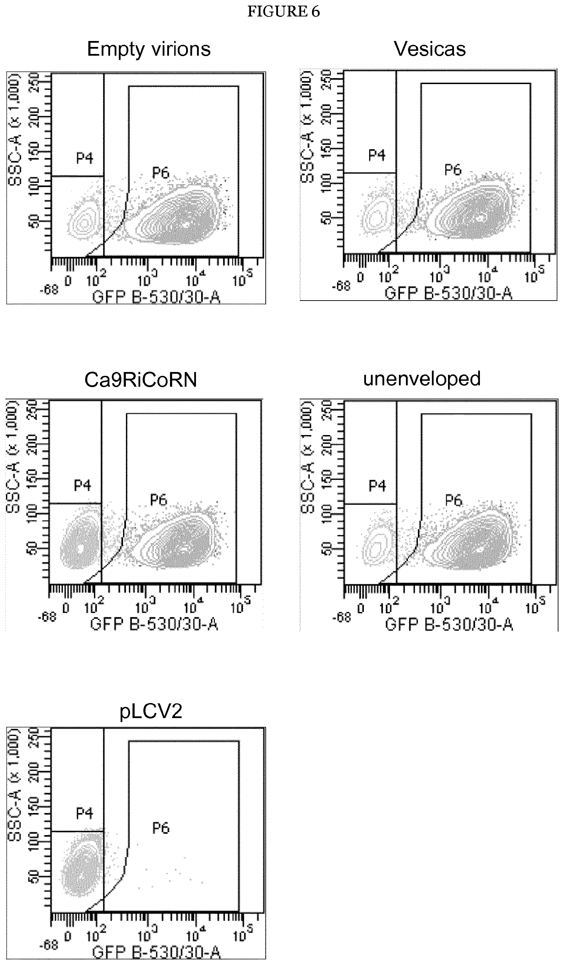

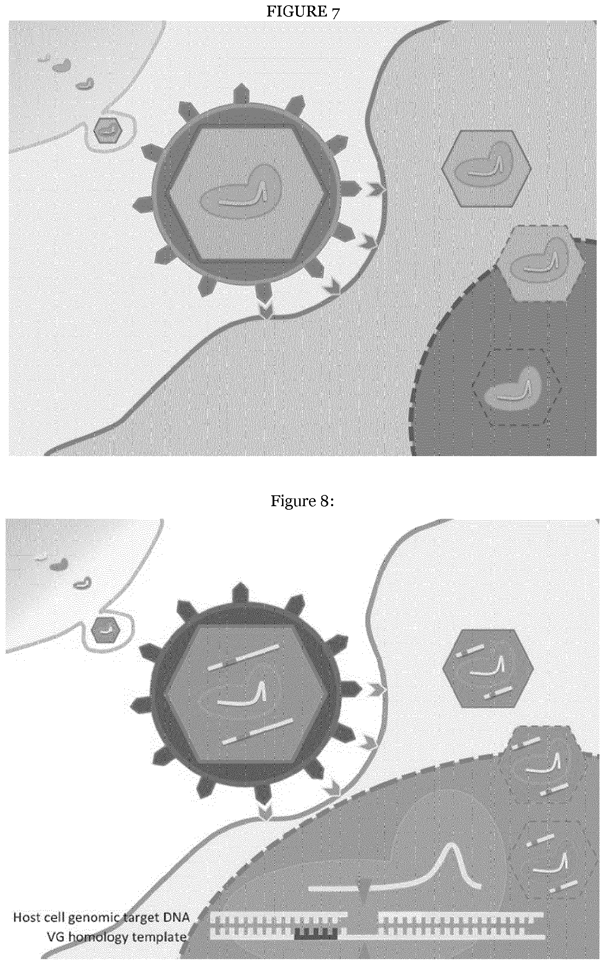

In order to find out whether it is possible to load CRISPR / Cas9 protein with sgRNA already in the virus producing cells, and thus, to generate a viral protein-shuttle for functional CRISPR / Cas9 ribonucleoproteins (RNP), the inventors generated a construct for the combined expression of CRISPR / Cas9 protein and sgRNA against green fluorescent protein. Co-expression of the construct on the side of the packaging plasmids gag / pol and env in HEK293 lentiX virus producing cells resulted in the generation of 1.5x10⁷ transducing units (TU), which contained a genomic deletion of green fluorescent protien in 40% of our HEK293 eGFP + reporter cells in the Flow cytometry (Figure 6). The general pathway of the CRISPR/Case ribonucleoprotein shuttle is depicted in Figure 7.

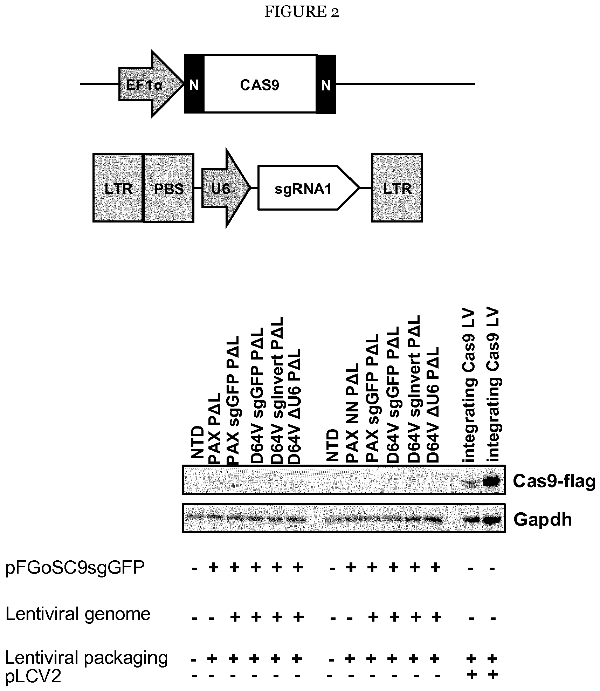

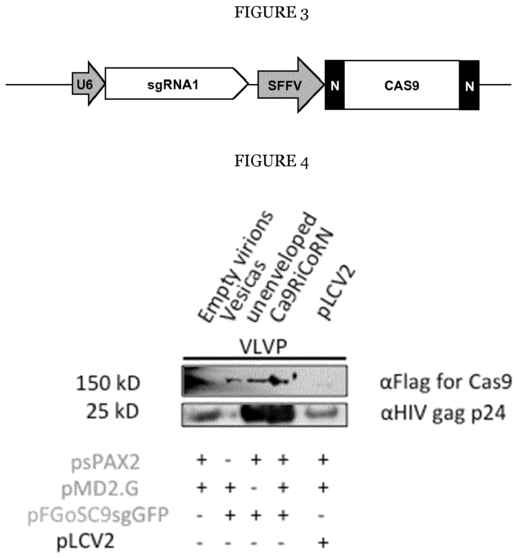

Compared with an application of CRISPR / Cas9 via integrating lentiviruses, a transient presence of CRISPR / Cas9 RNP in the recipient cells is demonstrated 12 hours after transduction with the CaPRiCoRN system the inventor's developed (Figure 2). According to the known half-life of CRISPR / Cas9 RNP of 24 hours, CaPRiCoRN transduced CRISPR / Cas9 RNP are no longer detectable in the target cells after 10 days (Figure 2). In addition, transduction of the target cells by exosomes can be ruled out since capsids with CRISPR / Cas9 RNP were not infectious in the absence of a viral envelope (Figure 4, Figure 6). A preparation of vesicles analogous to Montagna et al. ^{[8]} has at best a minimal efficiency with respect to a genomic deletion of GFP whereas the herein described CaPRiCoRN transduction system is capable of performing a substantial genetic deletion of GFP (Figure 4, Figure 6).

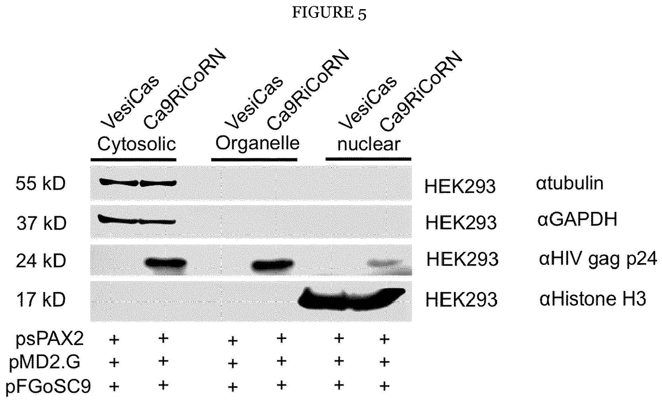

This dramatic increase in efficiency compared to the system of Montagna et al. ^{[8]} is generated by the co-transfection of the production cells with the psPAX2 plasmid, which among other things encodes the components of the viral capsid. The detection of CRISPR / Cas9 ribonucleoprotein in un-encapsulated capsids suggests that the increase in efficiency is due to mechanisms known from the natural life cycle of the lentivirus. (Figure 4). Accordingly, localization within the viral capsid protects CRISPR / Cas9 ribonucleoproteins from proteolytic or proteasomal degradation in the target cell. Consistent with literature ^{[9]}, the detection of p24 capsid antigens in nuclear fractionation suggests that the transduction system reported here is a direct nuclear shuttle for CRISPR-Cas9 ribonucleoproteins (Figure 5).

Besides editing capabilities for single base exchanges and the generations as well as corrections of frameshifts, the CaPRiCoRN system of the invention can also be used to precisely repair more complex and larger deletions or insertions: The inventors demonstrated that a RNA lentiviral genome is not necessary for incorporation of Cas9 RNPs into retroviral particles and hence can be omitted. By supplying the original Ca9RiCoRN system with a lentiviral genome which serves as a repair template flanked by 5' as well as 3' homology arms (template nucleic acid of the invention) and using for example a psPAX2D64V plasmid for the generation of integrase deficient lentiviral particles the ability of the system to perform the above mentioned precise edits is demonstrated (figure 8).

### Materials and Methods:

### Generation of a reporter cell line and cell culture:

All cell lines were obtained from ATCC. HEK 293 cells were cultured in DMEM supplemented with 10% FBS, 1%Penicillin/Streptomycin and 1% Sodium Pyruvate.. Reporter lines were generated using transduction with integrating lentiviral vectors coding for the expression of eGFP under control of SFFV promoter.

### Production of lentivirus-like Particles containing CRISPR/Cas9 ribonucleoparticles and transduction of cells:

A co/expression construct for CRISPR/Cas9 under control of an SFFV promoter and sgRNA controlled by U6 promoter was generated from pLentiCRISPRV2 plasmid (Addgene #52961, a gift from Feng Zhang) by removal of CMV viral promoter as well as viral LTRs and a promotor exchange from EFIα short promotor to SFFV. HEK 293 LentiX viral production cells (Takara Biotech, Kusatsu, Japan) were co-transfected using Polyethylenimine with the aforementioned construct alongside with packaging plasmids pMD2.G (addgene #12259, a gift from Didier Trono) coding for VSVG and psPAX2 (addgene#12260 a gift from Didier Trono) encoding viral proteins gag/pol in a 3:1:1 molar ratio. Viral supernatant was harvested after 72 hours and concentrated by sucrose cushion ultracentrifugation (20% w/v) at 50.000g for 02.30 hours at 4 degrees Celsius in a Beckman Coulter Optima XE (Pasadena, US). Viral titer was assessed using one-wash p24 ELISA kit (OriGene, Rockwell, US). HEK293 eGFP+ reporter cell lines were transduced with an MOI of 1,5x107 transducing units. For protein analysis an additional viral prep was performed using plasmids pLenti guide puro (addgene #52961, a gift from Feng Zhang) and hCas9 (addgene# 41815, a gift from George Church).

### Protein Analysis:

To assess protein levels of flag tagged-CRISPR/Cas9 in lentivirus like particels, supernatants from HEK293 LentiX virus producing cells were centrifuged by 1500 x g for 5 minutes to pellet cellular debris and supernatant was filtered through 0.45 µm filters to remove cellular components followed by sucrose cushion ultracentrifugation at 50.000g for 02:30 hours. To assess protein levels of flag tagged-CRISPR/Cas9 from transduced reporter cell lines, cells were washed twice in ice cold PBS before lysis. Protein was extracted using SDS Buffer (100 mM Tris-HCl pH 8, 150 mM NaCl ,10 mM EDTA pH 8 , 10% SDS). Homogenates were lysed for 30 min on the shaker at 25°C prior to clarification at 15.000 x g for 15 min at 4°C. Nuclear fractionation was performed according to the method of Holden and colleagues . published in BMC Res Notes in 2009.

Protein concentrations were determined by Pierce BCA Protein Assay kit (Pierce, Rockford, IL). 30 µg of protein were loaded onto 10% BIS-TRIS-Page gels and transferred to nitrocellulose membranes following electrophoresis. Membranes were blocked in 5% Non-fat dried milk and incubated with primary antibodies in appropriate dilutions overnight. Following incubation with HRP-conjugated secondary antibodies, the protein expression was visualized with a LI-COR chemiluminiscence detection system (Li-Cor, Lincoln, US).

### Flow cytometry:

Cells were washed twice in PBS and centrifugated at 500 rpm for 5 minutes to remove dead cells prior to flow cytometry. Reporter cell lines were directly subjected to flow cytometry for analysis of gene editing efficiency. Flow cytometry was performed on BD Fortessa (Beckton Dickinson, New Jersey, US)

### Reagents and antibodies:

flag M2 antibody (#F1804) was obtained from Sigma Aldrich (Sigma Aldrich, St.Louis, MS, US); Cas9 antibody (#12HCLC #711061) was obtained from Thermo fischer (Thermo Fischer, Waltham, MS, US); Hiv p24 gag (#MAB7360) was obtained from R&D Systems (R&D Systems, Minneapolis, MN, US).

### REFERENCES

The references are:
1. Cong, L. et al. Multiplex genome engineering using CRISPR/Cas systems. Science 339, 819-823 (2013).
2. Hacein-Bey-Abina, S. et al. A serious adverse event after successful gene therapy for X-linked severe combined immunodeficiency. N Engl J Med 348, 255-256 (2003).
3. Nightingale, S.J. et al. Transient gene expression by nonintegrating lentiviral vectors. Mol Ther 13, 1121-1132 (2006).
4. Chew, W.L. et al. A multifunctional AAV-CRISPR-Cas9 and its host response. Nat Methods 13, 868-874 (2016).
5. Ran, F.A. et al. In vivo genome editing using Staphylococcus aureus Cas9. Nature 520, 186-191 (2015).
6. Kim, S., Kim, D., Cho, S.W., Kim, J. & Kim, J.S. Highly efficient RNA-guided genome editing in human cells via delivery of purified Cas9 ribonucleoproteins. Genome Res 24, 1012-1019 (2014).
7. Choi, J.G. et al. Lentivirus pre-packed with Cas9 protein for safer gene editing. Gene Ther 23, 627-633 (2016).
8. Montagna, C. et al. VSV-G-Enveloped Vesicles for Traceless Delivery of CRISPR-Cas9. Mol Ther Nucleic Acids 12, 453-462 (2018).
9. Matreyek, K.A. & Engelman, A. Viral and cellular requirements for the nuclear entry of retroviral preintegration nucleoprotein complexes. Viruses 5, 2483-2511 (2013).
10. Adli M. The CRISPR tool kit for genome editing and beyond. Nature comm 9:1911 (2018)
11. Sanjana NE et al. Nat Methods. 2014 Aug; 11(8): 783-784.

## Claims

1. **A viral particle** for delivering cargo into a cell and/or into a cell nucleus, the viral particle comprising a first particle coat encasing a first particle volume, wherein the first particle volume comprises the cargo compound, and a shuttle compound, the shuttle compound comprising a gene-editing nuclease protein, or a fragment or derivative thereof.

2. The viral particle according to claim 1, wherein the cargo compound is the shuttle compound, or wherein the cargo compound is selected from a nucleic acid, a protein, a lipid, a small molecule, a macromolecule, a sugar, or combination thereof, and preferably the cargo compound is a component or a combination of components suitable for inducing a genome editing reaction in a target genome, such as a component or combination of components of the CRISPR/Cas9 genome editing system.

3. The viral particle according to claim 1 or 2, wherein the first particle coat is a viral capsid.

4. The viral particle according to claim 1 to 3, wherein the gene editing nuclease protein is not fused to a structural component of the first particle coat, preferably wherein such structural component is a protein, such as a viral coat protein.

5. The viral particle according to any one of claims 1 to 4, wherein the first particle volume further comprises at least one species of a gene-editing guide nucleic acid, such as a guide RNA (gRNA), or single guide RNA (sgRNA), or a guide DNA (gDNA) or single guide DNA (sgDNA) or combinations or variants thereof.

6. The viral particle according to any one of claims 1 to 5, further comprising at least one species of template nucleic acid, preferably having a 5' and 3' flanking region sequences homologous to sequences flanking the target sequence recognized by one or more gRNAs, and where preferably the template sequence is intended to be introduced.

7. The viral particle according to claim 5 or 6, wherein the gRNA is bound to the gene editing nuclease to form a ribonucleoprotein gene editing complex comprising a gene editing nuclease covalently or non-covalently bound to an RNA.

8. The viral particle according to any one of claims 1 to 7, wherein the viral particle is a retrovirus derived particle, preferably a lentivirus derived particle, and optionally comprises a viral particle surface-presented targeting molecule suitable for targeting the delivery of the viral particle to a target cell type.

9. **A method for producing a viral particle** for use in the delivery of a cargo compound into a cell and/or into a cell nucleus, the method comprising the step of
a) Introducing into a viral packaging cell one or more genetic constructs for the expression of a shuttle compound and a cargo compound, wherein the one or more genetic constructs comprise (i) a first expressible sequence encoding a gene-editing nuclease protein, or a derivative or fragment thereof, and (ii) a second expressible sequence encoding a cargo compound; alternatively, wherein (i) is not introduced into the viral packaging cell but provided as a stable, preferably genome integrated, expression construct in said viral packaging cell
b) Expressing the first and the second expressible sequences in the viral packaging cell,
c) Expressing viral particle components for viral packaging in the viral packaging cell and thereby packaging the shuttle compound and the cargo compound into the viral particle,
d) Harvesting the so produced viral particle.

10. **A method for producing a viral particle** for use in the delivery of gene-editing components into a target cell and/or into a target cell nucleus, the method comprising the step of
a) Introducing into a viral packaging cell one or more genetic constructs for the expression of gene-editing components, wherein the one or more genetic constructs comprise (i) a first expressible sequence encoding a gene-editing nuclease, or a derivative or fragment thereof and (ii) a second expressible sequence encoding at least for one gene-editing guide nucleic acid; alternatively, wherein (i) is not introduced into the viral packaging cell but provided as a stable, preferably genome integrated, expression construct in said viral packaging cell;
b) Expressing the first and the second expressible sequences in the viral packaging cell,
c) Expressing viral particle components for viral packaging in the viral packaging cell,
d) Harvesting the so produced viral particle.

11. The method according to claim 9 or 10, wherein the one or more genetic constructs and the packaging cell do not comprise a functional integrase, preferably viral integrase, more preferably lentiviral integrase.

12. **A composition** comprising a viral particle according to any one of claims 1 to 8, together with a carrier and/or excipient, preferably wherein the composition is pharmaceutical composition.

13. **A product for use in the treatment of a disease,** wherein the product is a viral particle according to any one of claims 1 to 8, or is a composition according to claim 12.

14. **An *in vitro* use** of (i) a viral particle according to any one of claims 1 to 8, or (ii) of a composition according to claim 12, in the targeted delivery of one or more cargo compounds into a target cell and/or into a target cell nucleus, or for use in the gene editing of a target cell.

15. **A method of editing a genomic sequence in a target cell,** comprising a step of contacting (or transducing) the target cell and/or a target cell nucleus, with a viral particle according to any one of claims 1 to 8, or with a composition according to any claim 12.

16. **A system for editing a target genome,** the system comprising
(i) A genetic expression construct comprising an expressible sequence of a gene-editing nuclease, or a fragment or derivative thereof,
(ii) A genetic expression construct comprising an expressible expression cassette for the introduction of a sequence of a gene-editing guide nucleic acid sequence,
wherein the genetic construct of (i) an (ii) can be provided on one nucleic acid molecule or separate nucleic acid molecules, and
wherein the genetic construct(s) is(are) not able to integrate into a target genome.


SEQUENCE LISTING

<110>  Johann Wolfgang Goethe-Universität Frankfurt am Main
       Deutsches Krebsforschungszentrum Stiftung des öffentlichen
       Rechts

<120>  METHODS AND PRODUCTS FOR A SAFE AND EFFICIENT TRANSDUCTION OF
       GENE EDITING COMPONENTS

<130>  U30891EP

<160>  2

<170>  PatentIn version 3.5

<210>  1
<211>  1368
<212>  PRT
<213>  Streptococcus pyogenes

<400>  1

<210>  2
<211>  1414
<212>  PRT
<213>  artificial

<220>
<223>  human codon optimized CRISPR-associated endonuclease Cas9/Csn1

<400>  2

Met Tyr Pro Tyr Asp Val Pro Asp Tyr Ala Ser Pro Lys Lys Lys Arg
1               5                   10                  15

Lys Val Glu Ala Ser Asp Lys Lys Tyr Ser Ile Gly Leu Asp Ile Gly
            20                  25                  30

Thr Asn Ser Val Gly Trp Ala Val Ile Thr Asp Glu Tyr Lys Val Pro
        35                  40                  45

Ser Lys Lys Phe Lys Val Leu Gly Asn Thr Asp Arg His Ser Ile Lys
    50                  55                  60

Lys Asn Leu Ile Gly Ala Leu Leu Phe Asp Ser Gly Glu Thr Ala Glu
65                  70                  75                  80

Ala Thr Arg Leu Lys Arg Thr Ala Arg Arg Arg Tyr Thr Arg Arg Lys
                85                  90                  95

Asn Arg Ile Cys Tyr Leu Gln Glu Ile Phe Ser Asn Glu Met Ala Lys
            100                 105                 110

Val Asp Asp Ser Phe Phe His Arg Leu Glu Glu Ser Phe Leu Val Glu
        115                 120                 125

Glu Asp Lys Lys His Glu Arg His Pro Ile Phe Gly Asn Ile Val Asp
    130                 135                 140

Glu Val Ala Tyr His Glu Lys Tyr Pro Thr Ile Tyr His Leu Arg Lys
145                 150                 155                 160

Lys Leu Val Asp Ser Thr Asp Lys Ala Asp Leu Arg Leu Ile Tyr Leu
                165                 170                 175

Ala Leu Ala His Met Ile Lys Phe Arg Gly His Phe Leu Ile Glu Gly
            180                 185                 190

Asp Leu Asn Pro Asp Asn Ser Asp Val Asp Lys Leu Phe Ile Gln Leu
        195                 200                 205

Val Gln Thr Tyr Asn Gln Leu Phe Glu Glu Asn Pro Ile Asn Ala Ser
    210                 215                 220

Gly Val Asp Ala Lys Ala Ile Leu Ser Ala Arg Leu Ser Lys Ser Arg
225                 230                 235                 240

Arg Leu Glu Asn Leu Ile Ala Gln Leu Pro Gly Glu Lys Lys Asn Gly

245                 250                 255

Leu Phe Gly Asn Leu Ile Ala Leu Ser Leu Gly Leu Thr Pro Asn Phe
            260                 265                 270

Lys Ser Asn Phe Asp Leu Ala Glu Asp Ala Lys Leu Gln Leu Ser Lys
        275                 280                 285

Asp Thr Tyr Asp Asp Asp Leu Asp Asn Leu Leu Ala Gln Ile Gly Asp
    290                 295                 300

Gln Tyr Ala Asp Leu Phe Leu Ala Ala Lys Asn Leu Ser Asp Ala Ile
305                 310                 315                 320

Leu Leu Ser Asp Ile Leu Arg Val Asn Thr Glu Ile Thr Lys Ala Pro
                325                 330                 335

Leu Ser Ala Ser Met Ile Lys Arg Tyr Asp Glu His His Gln Asp Leu
            340                 345                 350

Thr Leu Leu Lys Ala Leu Val Arg Gln Gln Leu Pro Glu Lys Tyr Lys
        355                 360                 365

Glu Ile Phe Phe Asp Gln Ser Lys Asn Gly Tyr Ala Gly Tyr Ile Asp
    370                 375                 380

Gly Gly Ala Ser Gln Glu Glu Phe Tyr Lys Phe Ile Lys Pro Ile Leu
385                 390                 395                 400

Glu Lys Met Asp Gly Thr Glu Glu Leu Leu Val Lys Leu Asn Arg Glu
                405                 410                 415

Asp Leu Leu Arg Lys Gln Arg Thr Phe Asp Asn Gly Ser Ile Pro His
            420                 425                 430

Gln Ile His Leu Gly Glu Leu His Ala Ile Leu Arg Arg Gln Glu Asp
        435                 440                 445

Phe Tyr Pro Phe Leu Lys Asp Asn Arg Glu Lys Ile Glu Lys Ile Leu
    450                 455                 460

Thr Phe Arg Ile Pro Tyr Tyr Val Gly Pro Leu Ala Arg Gly Asn Ser
465                 470                 475                 480

Arg Phe Ala Trp Met Thr Arg Lys Ser Glu Glu Thr Ile Thr Pro Trp
                485                 490                 495

Asn Phe Glu Glu Val Val Asp Lys Gly Ala Ser Ala Gln Ser Phe Ile
        500                 505                 510

Glu Arg Met Thr Asn Phe Asp Lys Asn Leu Pro Asn Glu Lys Val Leu
    515                 520                 525

Pro Lys His Ser Leu Leu Tyr Glu Tyr Phe Thr Val Tyr Asn Glu Leu
530                 535                 540

Thr Lys Val Lys Tyr Val Thr Glu Gly Met Arg Lys Pro Ala Phe Leu
545                 550                 555                 560

Ser Gly Glu Gln Lys Lys Ala Ile Val Asp Leu Leu Phe Lys Thr Asn
                565                 570                 575

Arg Lys Val Thr Val Lys Gln Leu Lys Glu Asp Tyr Phe Lys Lys Ile
            580                 585                 590

Glu Cys Phe Asp Ser Val Glu Ile Ser Gly Val Glu Asp Arg Phe Asn
        595                 600                 605

Ala Ser Leu Gly Thr Tyr His Asp Leu Leu Lys Ile Ile Lys Asp Lys
    610                 615                 620

Asp Phe Leu Asp Asn Glu Glu Asn Glu Asp Ile Leu Glu Asp Ile Val
625                 630                 635                 640

Leu Thr Leu Thr Leu Phe Glu Asp Arg Glu Met Ile Glu Glu Arg Leu
                645                 650                 655

Lys Thr Tyr Ala His Leu Phe Asp Asp Lys Val Met Lys Gln Leu Lys
            660                 665                 670

Arg Arg Arg Tyr Thr Gly Trp Gly Arg Leu Ser Arg Lys Leu Ile Asn
        675                 680                 685

Gly Ile Arg Asp Lys Gln Ser Gly Lys Thr Ile Leu Asp Phe Leu Lys
    690                 695                 700

Ser Asp Gly Phe Ala Asn Arg Asn Phe Met Gln Leu Ile His Asp Asp
705                 710                 715                 720

Ser Leu Thr Phe Lys Glu Asp Ile Gln Lys Ala Gln Val Ser Gly Gln
                725                 730                 735

Gly Asp Ser Leu His Glu His Ile Ala Asn Leu Ala Gly Ser Pro Ala
            740                 745                 750

Ile Lys Lys Gly Ile Leu Gln Thr Val Lys Val Val Asp Glu Leu Val
        755                 760                 765

Lys Val Met Gly Arg His Lys Pro Glu Asn Ile Val Ile Glu Met Ala
    770                 775                 780

Arg Glu Asn Gln Thr Thr Gln Lys Gly Gln Lys Asn Ser Arg Glu Arg
785                 790                 795                 800

Met Lys Arg Ile Glu Glu Gly Ile Lys Glu Leu Gly Ser Gln Ile Leu
                805                 810                 815

Lys Glu His Pro Val Glu Asn Thr Gln Leu Gln Asn Glu Lys Leu Tyr
            820                 825                 830

Leu Tyr Tyr Leu Gln Asn Gly Arg Asp Met Tyr Val Asp Gln Glu Leu
        835                 840                 845

Asp Ile Asn Arg Leu Ser Asp Tyr Asp Val Asp His Ile Val Pro Gln
    850                 855                 860

Ser Phe Leu Lys Asp Asp Ser Ile Asp Asn Lys Val Leu Thr Arg Ser
865                 870                 875                 880

Asp Lys Asn Arg Gly Lys Ser Asp Asn Val Pro Ser Glu Glu Val Val
                885                 890                 895

Lys Lys Met Lys Asn Tyr Trp Arg Gln Leu Leu Asn Ala Lys Leu Ile
            900                 905                 910

Thr Gln Arg Lys Phe Asp Asn Leu Thr Lys Ala Glu Arg Gly Gly Leu
        915                 920                 925

Ser Glu Leu Asp Lys Ala Gly Phe Ile Lys Arg Gln Leu Val Glu Thr
    930                 935                 940

Arg Gln Ile Thr Lys His Val Ala Gln Ile Leu Asp Ser Arg Met Asn
945                 950                 955                 960

Thr Lys Tyr Asp Glu Asn Asp Lys Leu Ile Arg Glu Val Lys Val Ile
                965                 970                 975

Thr Leu Lys Ser Lys Leu Val Ser Asp Phe Arg Lys Asp Phe Gln Phe
            980                 985                 990

Tyr Lys Val Arg Glu Ile Asn Asn  Tyr His His Ala His  Asp Ala Tyr
        995                 1000                1005

Leu Asn Ala Val Val Gly Thr Ala Leu Ile Lys Lys Tyr Pro Lys
    1010                1015                1020

Leu Glu Ser Glu Phe Val Tyr Gly Asp Tyr Lys Val Tyr Asp Val
    1025                1030                1035

Arg Lys Met Ile Ala Lys Ser Glu Gln Glu Ile Gly Lys Ala Thr
    1040                1045                1050

Ala Lys Tyr Phe Phe Tyr Ser Asn Ile Met Asn Phe Phe Lys Thr
    1055                1060                1065

Glu Ile Thr Leu Ala Asn Gly Glu Ile Arg Lys Arg Pro Leu Ile
    1070                1075                1080

Glu Thr Asn Gly Glu Thr Gly Glu Ile Val Trp Asp Lys Gly Arg
    1085                1090                1095

Asp Phe Ala Thr Val Arg Lys Val Leu Ser Met Pro Gln Val Asn
    1100                1105                1110

Ile Val Lys Lys Thr Glu Val Gln Thr Gly Gly Phe Ser Lys Glu
    1115                1120                1125

Ser Ile Leu Pro Lys Arg Asn Ser Asp Lys Leu Ile Ala Arg Lys
    1130                1135                1140

Lys Asp Trp Asp Pro Lys Lys Tyr Gly Gly Phe Asp Ser Pro Thr
    1145                1150                1155

Val Ala Tyr Ser Val Leu Val Val Ala Lys Val Glu Lys Gly Lys
    1160                1165                1170

Ser Lys Lys Leu Lys Ser Val Lys Glu Leu Leu Gly Ile Thr Ile
    1175                1180                1185

Met Glu Arg Ser Ser Phe Glu Lys Asn Pro Ile Asp Phe Leu Glu
    1190                1195                1200

Ala Lys Gly Tyr Lys Glu Val Lys Lys Asp Leu Ile Ile Lys Leu
    1205                1210                1215

Pro Lys Tyr Ser Leu Phe Glu Leu Glu Asn Gly Arg Lys Arg Met
    1220                1225                1230

Leu Ala Ser Ala Gly Glu Leu Gln Lys Gly Asn Glu Leu Ala Leu

1235                    1240                    1245

Pro Ser Lys Tyr Val Asn Phe Leu Tyr Leu Ala Ser His Tyr Glu
    1250                    1255                    1260

Lys Leu Lys Gly Ser Pro Glu Asp Asn Glu Gln Lys Gln Leu Phe
    1265                    1270                    1275

Val Glu Gln His Lys His Tyr Leu Asp Glu Ile Ile Glu Gln Ile
    1280                    1285                    1290

Ser Glu Phe Ser Lys Arg Val Ile Leu Ala Asp Ala Asn Leu Asp
    1295                    1300                    1305

Lys Val Leu Ser Ala Tyr Asn Lys His Arg Asp Lys Pro Ile Arg
    1310                    1315                    1320

Glu Gln Ala Glu Asn Ile Ile His Leu Phe Thr Leu Thr Asn Leu
    1325                    1330                    1335

Gly Ala Pro Ala Ala Phe Lys Tyr Phe Asp Thr Thr Ile Asp Arg
    1340                    1345                    1350

Lys Arg Tyr Thr Ser Thr Lys Glu Val Leu Asp Ala Thr Leu Ile
    1355                    1360                    1365

His Gln Ser Ile Thr Gly Leu Tyr Glu Thr Arg Ile Asp Leu Ser
    1370                    1375                    1380

Gln Leu Gly Gly Asp Lys Arg Pro Ala Ala Thr Lys Lys Ala Gly
    1385                    1390                    1395

Gln Ala Lys Lys Lys Lys Asp Tyr Lys Asp Asp Asp Asp Lys Gly
    1400                    1405                    1410

Ser